Europäisches Patentamt

European Patent Office

Office européen des brevets

(11) Publication number: **0 068 555**
**A1**

# EUROPEAN PATENT APPLICATION

(21) Application number: 82200733.2

(22) Date of filing: 14.06.82

(51) Int. Cl.³: **A 61 L 2/08, C 08 L 23/12**

(30) Priority: 26.06.81 IT 2258581

(43) Date of publication of application: 05.01.83 Bulletin 83/1

(84) Designated Contracting States: **BE CH DE FR GB LI NL**

(71) Applicant: **ANIC S.p.A., Via Ruggero Settimo, 55, I-90139 Palermo (IT)**

(72) Inventor: **Lenzi, Paolo, Viale Romagna 2, I-20075 Lodi (Milan) (IT)**

(74) Representative: **Roggero, Sergio et al, Ing. Barzanò & Zanardo Milano S.p.A. Via Borgonuovo 10, I-20121 Milano (IT)**

(54) **Method for manufacturing polypropylene articles, particularly hypodermic syringes, which can be sterilized by gamma rays without becoming brittle.**

(57) A method for manufacturing polypropylene articles, in particular hypodermic syringes, which can be sterilised by irradiation using gamma rays without becoming brittle, consisting of adding a small quantity of low density polyethylene, in practice between 1 and 8%, to the polypropylene used for manufacturing said articles.

EP 0 068 555 A1

This invention relates to a method for manufacturing polypropylene articles, in particular hypodermic syringes, which can be sterilised by irradiation using gamma rays, without losing their mechanical characteristics, in particular impact strength, which consists of adding a small quantity of low density polyethylene, in practice between 1 and 8% by weight, to the polypropylene.

Disposable polypropylene hypodermic syringes have been manufactured for some time. Polypropylene is preferred because of its non-toxicity, its inertia towards the drugs and towards the liquids and solvents used for the injectable packs, because of its rigidity, because of its ease of working by injection moulding, and not lastly because of its low cost. Thus in communities such as hospitals, surgeries, dispensaries etc., they have replaced glass syringes, which are more fragile, heavier and less practical because of their requirement of continuous sterilisation.

Polypropylene syringes obviously also require preliminary sterilisation so that they can be marketed in a sterile pack ready for use.

Generally, sterilisation is carried out by means of ethylene oxide. The gaseous ethylene oxide acts on the syrings already packed in its wrapper, as the wrappers are generally formed from coupled polyethylene/cellophane films or polyethylene films alone, which are themselves coupled to paper at the welding points, through which the ethylene oxide can

- 2 -

0068555

therefore penetrate.

This sterilisation method is however not totally reliable precisely because of the presence of paper at the welding points.

Sterilisation by means of gamma rays (obtained from radioactive cobalt or from a cobalt bomb) has therefore recently been used, this ensuring total, reliable and comfortable sterilisation because it can be carried out on syringes already packed in totally impermeable welded wrappers, and even those already boxed for transportation and storage. This thus ensures prolonged sterility, even for long storage times. This sterilisation can be more costly than that using ethylene oxide, but is certainly more reliable. However, treatment with gamma rays has negative effects on the polypropylene used for manufacturing the syringes. This is because it favours depolymerisation of the propylene and its crystalline restructurisation.

Thus on prolonged storage, the syringes become brittle, and therefore break more easily, in particular at the mouth of the needle.

It has now been found that by mixing a small quantity of low density polyethylene with the polypropylene to be used for the injection moulding, this drawback can be obviated. The polyethylene must be carefully and intimately mixed with the polypropylene before melting, so as to obtain a homogeneous mixture containing the two polymers intimately dispersed. This is done by the normal machinery (screw

extruders) used for mixing the auxiliaries such as dyes, stabilisers etc. into the plastics material, and for subsequent granulation.

It is probable, but this is only our interpretation, that the low density polyethylene represents an obstacle to the crystalline restructurisation of the polypropylene, by producing steric hindrance and thus preventing crystallisation. It is also possible that the gamma rays induce slight cross-linkage of the polyethylene, which would thus increase the mechanical strength of the manufactured article. The quantity of low density polyethylene is fairly critical, because below a certain level it has no influence on the polypropylene, whereas if more than 8% it gives a manufactured article which is too plastic, and in addition tends to come out of mixture because of its incompatability with the polypropylene.

Mixtures were prepared with low density polyethylene contents of 1, 3, 4, 5 and 8%, and various batches of syringes of different sizes were prepared from these.

1, 2, 5 and 10 cc syringes were moulded, and were then enclosed in polyethylene film wrappers which were finally welded and boxed.

They were then sterilised by gamma rays with doses exceeding 2.5 megarad.

After prolonged storage (more than 6 months), the syringes exhibited the same impact strength as syringes which had just been extruded. They suffered no fracture as the

- 4 -                                    0068555

result of repeated stresses, and even when crushed by weights or sufficient force, they exhibited a plastic fracture which was in contrast to syringes made only of polypropylene, which even after two months were more sensitive to stress and, when broken, exhibited a brittle fracture.

Of the various sets of samples prepared and sterilised, it was found that those produced from mixtures containing 1% of low density polyethylene exhibited a more brittle fracture and thus resisted for much less time (about four months) than those to which 4-5% of low density polyethylene had been added.

With higher added quantities, the polypropylene became too soft and lost the rigidity necessary for the purpose, losing a large part of its bending resistance. With additions exceeding 8%, a tendency for the two polymers to come out of mixture begins to appear.

This method is obviously applicable equally well to any other polypropylene article to be subjected to irradiation by gamma rays and of which the mechanical characteristics must be safeguarded even after a prolonged time.

CLAIMS:

1. A method for manufacturing polypropylene articles, in particular hypodermic syringes, which can be sterilised by irradiation with gamms rays without alteration of their mechanical characteristics, in particular impact strength, even during prolonged ageing, consisting of adding a quantity of low density polyethylene of between 1 and 8% to the polypropylene.

2. A method as claimed in claim 1, wherein the added quantity of low density polyethylene is between 4 and 5%.

3. Articles which can be sterilised by gamms rays, comprising polypropylene mixed with between 1 and 8% of low density polyethylene.

European Patent
Office

**EUROPEAN SEARCH REPORT**

0068555

Application number

EP 82 20 0733

## DOCUMENTS CONSIDERED TO BE RELEVANT

| Category | Citation of document with indication, where appropriate, of relevant passages | Relevant to claim | CLASSIFICATION OF THE APPLICATION (Int. Cl. 3) |
|---|---|---|---|
| X | GB-A-1 422 454 (CHISSO CORPORATION) *Claims 1,8; page 1, lines 90-95* | 1-3 | A 61 L 2/08<br>C 08 L 23/12 |
| A | DE-A-1 569 429 (VEBA-CHEMIE) *Claim; page 2, lines 1-28* | 1-3 | |

TECHNICAL FIELDS
SEARCHED (Int. Cl. 3)

C 08 L
A 61 L

The present search report has been drawn up for all claims

| Place of search | Date of completion of the search | Examiner |
|---|---|---|
| THE HAGUE | 06-10-1982 | GOOVAERTS R.E. |